# EUROPEAN PATENT APPLICATION

(11) **EP 3 878 969 A1**
(43) Date of publication of application: **15.09.2021**
(21) Application number: 19881419.6
(22) Date of filing: 08.11.2019
(51) Int. Cl.: C12Q 1/68, G01N 1/28

(54) **SAMPLE SLIDE FOR GENETIC TEST**

(30) Priority: 09.11.2018 JP 2018211629
(71) Applicant: LSI Medience Corporation, Tokyo 101-8517 (JP)
(72) Inventor: SHIMADZU Mitsunobu, Tokyo 101-8517 (JP); WAKAMATSU Hirotake, Tokyo 101-8517 (JP)
(74) Representative: Gille Hrabal Partnerschaftsgesellschaft mbB Patentanwälte
(86) International application number: PCT/JP2019/043809
(87) International publication number: WO 2020/096031

(57) **Abstract**

Provided is a specimen slide for genetic testing, by which: (1) a step for scraping off an FFPE section from an unstained slide can be simplified without affecting the subsequent nucleic acid extraction step; (2) the risk of contamination when scraping off can be avoided; and (3) a cancer site can be selectively separated by an inexpensive and simple means. The specimen slide for genetic testing is a specimen slide for genetic testing on which a specimen section is to be mounted, wherein part of the slide can be separated, together with the specimen section to be separated, from the rest of the slide, while maintaining an upper surface and a lower surface of the slide.

SEQUENCE LISTING

<110>  LSI Medience Corporation

<120>  Specimen slide for genetic testing

<130>  LSI-042

<150>  JP 2018-211629
<151>  2018-11-09

<160>  3

<170>  PatentIn version 3.5

<210>  1
<211>  22
<212>  DNA
<213>  artificial sequence

<220>
<223>  Description of Artificial Sequence: F primer

<400>  1
cctatgactt cttgtgcatt gc                                                22

<210>  2
<211>  20
<212>  DNA
<213>  artificial sequence

<220>
<223>  Description of Artificial Sequence: R primer

<400>  2
tacacctctg ggaactggtc                                                   20

<210>  3
<211>  21
<212>  DNA
<213>  artificial sequence

<220>
<223>  Description of Artificial Sequence: Probe

<400>  3
gctgccatcc ggaagaaact g                                                 21

## Description

### TECHNICAL FIELD

The present invention relates to a specimen slide for genetic testing.

### BACKGROUND ART

Although various test materials (specimens) are handled in genetic testing, cancer companion diagnostics have appeared, and the most common test material at present is unstained slides (slide specimens in which formalin-fixed paraffin-embedded (FFPE) sections are fixed on microscope slides). When extracting nucleic acid from an unstained slide, the FFPE section is scraped from the microscope slide with a scalpel, and the scraped FFPE section is placed in a plastic microtube to extract the nucleic acid. If the tissue size per slide is small (needle biopsy), since sufficient nucleic acid cannot be extracted, it is necessary to scrape FFPE sections from multiple slides per specimen. By contrast, if a large number of specimens are treated, it is a very complicated work and requires a lot of labor. Furthermore, the act of putting the scraped PPFE section into a microtube is extremely difficult and requires skill due to the influence of static electricity. Furthermore, there is also a risk of contamination between specimens due to scattering of scraped FFPE sections.

Additionally, cancer testing requires the presence of the cancer site in the slide specimen of the test material. Therefore, when preparing a specimen, continuous sections are prepared, and HE staining is carried out using one of the sections, and the pathologist confirms the presence of the cancer site. By selectively examining only the tumor tissue, it is possible to avoid the influence of the surrounding normal tissues and obtain more accurate test results. For specimens with a high proportion of normal sites, it is necessary to mark the cancer site to indicate the area to be examined. Therefore, the area equivalent to the marking may be scraped from the unstained slide with reference to the specimen marked by HE staining.

The laser microdissection method is known as a method of scraping part of this specimen, which is a method of attaching a special film to a tissue and cutting it with a laser. This method has the following problems. That is to say, this method requires special equipment, and is expensive; this method requires a special microscope slide or plastic film for cutting with a laser, and is expensive; and it takes time to selectively cut out from one slide. This method is not suitable for clinical tests that process many specimens.

In recent years, a tissue dissection method (Specimen Pretreatment Apparatus "AVENIO Millisect", Roche Diagnostics, Non-patent literature 1) was developed for testing by shortening the cutting time compared to the laser microdissection method. This apparatus uses a dedicated tip equipped with a plastic blade (cutting blade) at the bottom of the tip and a die section sample collection chamber inside the tubular body, and brings the tip into contact with a microscope slide to which an FFPE section is mounted. The section on the slide is physically cut out by rotating the chip, and the fragments of the section are collected in the die section sample collection chamber. Since the chip used in the apparatus are expensive (approximately 4000 yen/tip) and are disposable to prevent contamination, there is a price problem in clinical tests that process many specimens.

### CITATION LIST

### NON-PATENT LITERATURES

[Non-patent literature 1] "AVENIO Millisect" Package Insert, Prepared: August, 2017 (1st version)

### SUMMARY OF INVENTION

### TECHNICAL PROBLEM

Therefore, an object of the present invention is to provide a method of pretreating a specimen, by which: (1) a step for scraping off an FFPE section from an unstained slide can be simplified without affecting the subsequent nucleic acid extraction step, (2) the risk of contamination when scraping off can be avoided, and (3) a cancer site can be selectively separated by an inexpensive and simple means; and a medical instrument and an apparatus used for the pretreatment method.

### SOLUTION TO PROBLEM

The problems can be solved by the following invention:
[1] A specimen slide for genetic testing on which a specimen section is to be mounted, wherein part of the slide can be separated, together with the specimen section to be separated, from the rest of the slide, while maintaining an upper surface and a lower surface of the slide.
[2] A specimen slide for genetic testing on which a specimen section is to be mounted, wherein a guide that assists in separating part of the slide from the rest of the slide is disposed on a surface on which the specimen section is to be mounted and/or an opposite surface thereof.
[3] A specimen slide for genetic testing on which a specimen section is to be mounted, wherein a groove that assists in separating part of the slide from the rest of the slide is disposed on an opposite surface of a surface on which the specimen section is to be mounted.
[4] A system for pretreating a specimen, said system comprising:
   an apparatus for pretreating a specimen equipped with a separator capable of separating part of the specimen slide for genetic testing according to any one of claims 1 to 3 from the rest of the slide; and
   the specimen slide for genetic testing according to any one of claims 1 to 3.
[5] A method of pretreating a specimen, said method comprising:
   (1) preparing a specimen section;
   (2) mounting the specimen section on a slide; and
   (3) separating part of the slide, together with the specimen section mounted on the area, from the rest of the slide.
[6] A method of pretreating a specimen, said method comprising:
   (1) preparing a paraffin-embedded specimen section;
   (2) mounting the specimen section on a slide;
   (3) separating part of the slide, together with the specimen section mounted on the area, from the rest of the slide; and
   (4) removing paraffin from the specimen section on the separated slide.

### ADVANTAGEOUS EFFECTS OF INVENTION

According to the present invention, a step for scraping off an FFPE section from an unstained slide can be simplified without affecting the subsequent nucleic acid extraction step. Furthermore, the risk of contamination when scraping off can be avoided. Furthermore, a cancer site can be selectively separated by an inexpensive and simple means.

### BRIEF DESCRIPTION OF THE DRAWINGS

[Fig. 1] Fig. 1 is photographs, instead of drawings, showing the state in which an FFPE section is fixed on a polystyrene (PS) slide. The left side shows the state of the PS slide (before fixing the FFPE section) with 7 mm x 7 mm grid-like grooves, and the right side shows the state of the PS slide after fixing the FFPE section.
[Fig. 2] Fig. 2 is a photograph, instead of a drawing, showing the electric heating cutter used in Example 2.
[Fig. 3] Fig. 3 is a photograph, instead of a drawing, showing how the PS slide is cut along the grooves while cooling the underside of the PS slide.
[Fig. 4] Fig. 4 is photographs, instead of drawings, showing the states before and after cutting the PS slide
[Fig. 5] Fig. 5 is a photograph, instead of a drawing, showing the states before and after deparaffinization (step (4) in Example 3).
[Fig. 6] Fig. 6 is a photograph and graphs showing the results of absorbance measurement to determine the amount of DNA extracted for each area when extracted from 2 slide pieces of 7 mm × 7 mm. From the left, the graphs show Area (2-AB), Area (3-AB), Area (4-AB), and Area (5-AB).
[Fig. 7] Fig. 7 is a photograph and graphs showing the results of absorbance measurement to determine the amount of DNA extracted for each area when extracted from 3 slide pieces of 7 mm × 7 mm. From the left, the graphs show Area (2-ABC), Area (3-ABC), Area (4-ABC), and Area (5-ABC).

### DESCRIPTION OF EMBODIMENTS

In the present invention, a specimen section sliced from a tissue specimen or a cell specimen (in particular, a pathological tissue/cell specimen), preferably a formalin-fixed paraffin-embedded tissue specimen or cell specimen, is mounted on the slide of the present invention; only a necessary area, such as a cancer site, is separated from the rest of the specimen section, together with part of the slide that corresponds to the area, using an appropriate means; and then, a nucleic acid to be used for genetic testing is extracted from the specimen section on the separated slide.

The specimen that can be handled in the present invention is not particularly limited, but a general specimen for carrying out pathological diagnosis can be widely handled. Examples of the specimen include tissue specimens, cell specimens, and the like. Preferably, it is a formalin-fixed paraffin-embedded tissue specimen or cell specimen (FFPE tissue specimen or cell specimen) by a conventional method.

In the present invention, a specimen section obtained by slicing the specimen by a conventional method (for example, a microtome) is spread and mounted on a surface, on which the specimen section is to be mounted, of the slide of the present invention.

The shape and size of the slide of the present invention are not particularly limited, but it is preferable that the shape and size are similar to those of conventionally known microscope slides for preparing observation specimens so that the operation feeling is the same as before and existing instruments can be used. For example, it has a rectangular shape with a length of approximately 76 mm, a width of approximately 26 mm, and a thickness of approximately 1 mm.

In the slide of the present invention, at least one surface is a mounting surface on which a specimen section is to be mounted, and part of the slide can be separated, together with the specimen section to be separated, from the rest of the slide. In the present invention, a guide(s) that assists in separation is disposed on the mounting surface (i.e., the surface on which the specimen section is to be mounted) and/or the opposite surface thereof (hereinafter referred to as the basal surface) so that, when part of the slide is separated from the rest of the slide, they can be separated while maintaining the upper surface and the lower surface of the slide. The guide(s) may be disposed on either one or both of the mounting surface or the basal surface of the slide, but it is preferable to dispose the guide on the basal surface so as not to damage the specimen section during separation.

Since the specimen section is placed on the mounting surface, it is preferable that the area on which the specimen section is placed (hereinafter referred to as the placement area) is a smooth surface. In connection with this, areas not intended to place the specimen section, such as the edges of the slide, need not be smooth. For example, it can also be roughened to prevent slipping when held, or it is also possible to provide a memo field suitable for describing information.

Furthermore, the mounting surface is preferably hydrophilic so that the paraffin-embedded specimen section can easily adhere to it. For example, the entire slide can also be made from a hydrophilic material, or hydrophilic treatment can also be applied to the surface to be the mounting surface of the slide made of any material. Of the entire slide surface to be the mounting surface, it is possible to make it easier to adhere to the desired place by making the parts other than the part suitable for mounting hydrophobic or water-repellent.

The guide provided on the mounting surface and/or the basal surface in the slide of the present invention is not particularly limited, as long as it can assist in separating part of the slide from the rest of the slide. For example, lines that serve as a mark and grooves that can reduce the force required for separation can be exemplified. The slide of the present invention is characterized by separating the specimen section together with the slide area on which the specimen section is placed in order to selectively separate only the required area (for example, a cancer site) of the specimen section mounted on the mounting surface. As the separation method in the present invention, for example, a method of punching only the required area with a punching tool, a method of cutting out only the required area by incision means, a method of separating only the required area by incision means, a method of applying force to the slide to bend and separate the required area from the unnecessary area, or the like, can be exemplified.

Hereinafter, the present invention will be explained with reference to an embodiment in which grooves are provided as the guide, but the explanations can be appropriately applied for those skilled in the art to embodiments other than the grooves (for example, lines that serve as a marker). As the pattern of grooves provided on the basal surface of the slide, for example, grooves can be provided so that areas with a predetermined shape are continuously arranged. For example, if the area has a hexagonal shape, grooves can be provided so that the hexagons are arranged in a honeycomb shape. If the area has a square shape, grooves can be provided so that the squares are arranged in a grid pattern. The other shape is not particularly limited as long as the inside of the placing area is filled without gaps when viewed from the mounting surface of the slide. Examples of the shape include a triangular shape, a quadrangular shape (for example, square, rectangle, parallelogram, trapezoid, rhombus, or the like), or combinations thereof. In the case of such a continuous arrangement, any one or more areas can be separated (for example, punching, cutting out, separating by cutting, bending, or the like), so that there is an advantage that a desired area in the placing area can be separated.

As another pattern of grooves provided on the basal surface of the slide, for example, grooves can be provided so that areas with a predetermined shape are discontinuously arranged (that is, the predetermined shapes are arranged like islands floating in the sea). As the shape in this case, any shape can be adopted in addition to the above-mentioned shapes for continuous arrangement, and examples of the shape include a circular shape (perfect circle or ellipse) or the like. In the case of such a discontinuous arrangement, there is an advantage that any shape can be adopted.

As yet another pattern of grooves provided on the basal surface of the slide, for example, grooves can be provided so that multiple straight lines parallel to the short side or long side (preferably the short side) of the slide are arranged at arbitrary intervals (preferably even intervals), or so that only one straight line parallel to the short side or long side (preferably the short side) of the slide are arranged. In this case, since the required area can be separated from the unnecessary area by applying force to the slide and bending, there is an advantage that no special equipment is required.

The depth and width of the groove provided on the basal surface of the slide are not particularly limited, as long as the force required for separating part of the slide from the rest of the slide can be reduced, and can be appropriately determined depending on, for example, the thickness of the slide, the material, the force and method for separation, or the like.

The depth of the groove is, for example, 25% or more, preferably 50% or more, and more preferably 75% or more with respect to the thickness of the slide. If the groove is too deep, the strength of the slide may not be sufficient, and the groove depth is preferably 90% or less with respect to the thickness of the slide.

The width of the groove is not particularly limited, as long as the groove can be formed on the slide and the slide can be handled. Since if the width of the groove is too large, the selectivity will decrease, it is preferable that the width is 1 mm or less. Furthermore, it is preferable that the width is 0.1 mm or more in consideration of the selectivity for the groove when punching or the like. It is preferable that the width is approximately 0.5 mm in consideration of the ease of separation.

In the grooves provided on the basal surface of the slide, the spacing between adjacent grooves can be set according to the purpose of analysis. Considering the selectivity of tumor tissue or the like, in the case of a rectangular shape, the spacing is, for example, 10 mm or less in length and 10 mm or less in width, preferably 5 mm or less in length and 5 mm or less in width, and more preferably approximately 2 mm × 2 mm. The lower limit is not particularly limited and is, for example, 0.5 mm × 0.5 mm or more. Those skilled in the art can appropriately design the spacing in consideration of possible separation, and the ease of handling after separation by cutting and the width of the groove.

Depending on the spacing required for analysis, the size of the separation can be appropriately selected from the adjacent multiple grooves.

The material of the slide of the present invention is not particularly limited, as long as part of the specimen and the slide can be separated from the rest thereof in a state of mounting the specimen on the mounting surface of the slide, and the nucleic acid extraction after separation is not affected. Examples of such material include glass, resins, or the like. As the resins, organic resins (for example, polyacrylic, polyamide, polybutylene phthalate, polycarbonate, polyethylene, polyethylene phthalate, polyacetal, polypropylene, polyphenylene oxide, polyphenylene sulfide, polystyrene, polyvinyl chloride, ABS resin, AS resin, chlorotrifluoroethylene, fluoride vinylidene, perfluoroalkoxy alkane, or the like) or the like are exemplified. For example, glass, polypropylene, polystyrene, polyethylene, polyacrylic, or the like, which are generally used in the field of gene analysis, are most preferable, and when transparency is required for microscopic observation or the like, glass, polyacrylic, or the like is preferable.

Although a material capable of mounting the specimen section on the mounting surface of the slide is preferable, even if it is a material that cannot mount the specimen section on the mounting surface of the slide, any material that can impart such a characteristic by an appropriate surface treatment (for example, hydrophilic treatment) can be used. For example, when a cell tissue section is generally fixed on a microscope slide, the negative charge of phospholipids on the cell surface can be used for adhesion. Therefore, the surface of the microscope slide can be coated with an amino acid, such as poly-L-lysine or aminosilane, in order to improve the adhesiveness. Furthermore, considering the workability of spreading cell tissue pieces, it is efficient to have a hydrophilic surface.

The slide of the present invention can be combined with an apparatus for pretreating a specimen equipped with a predetermined separator to form a system for pretreating a specimen of the present invention.

The separator in the apparatus for pretreating a specimen is not particularly limited, as long as part of the slide of the present invention can be from the rest of the slide.

When the separation is carried out by punching, as the separator, a punching tool capable of punching only a required area can be exemplified.

When the separation is carried out by cutting out or separation by cutting, as the separator, a cutting means capable of cutting out or separating only a required region can be exemplified.

When the separation is carried out by bending, as the separator, a bending means capable of bending and separating the required area from the unnecessary area can be exemplified.

A pretreatment method of the present invention comprises:
(1) preparing a specimen section (preferably a paraffin-embedded specimen section);
(2) mounting the specimen section on a slide; and
(3) separating part of the slide, together with the specimen section mounted on the area, from the rest of the slide.
   When the specimen is a paraffin-embedded specimen section, a pretreatment method of the present invention comprises, in addition to the steps (1) to (3),
(4) removing paraffin from the specimen section on the separated slide.

In the step (1) of preparing a specimen section and the step (2) of mounting a specimen section in the method of the present invention, a specimen section can be prepared and mounted on a slide (preferably the slide of the present invention) in the same manner as in normal observation specimen preparation. For example, when a formalin-fixed paraffin-embedded specimen section (a FFPE specimen section) is used as the specimen section, specimen sections can be obtained by, for example, embedding a tissue specimen or a cell specimen collected from a patient or the like with formalin, carrying out each operation of dehydration, degreasing, paraffin penetration, and paraffin embedding, and continuously slicing the obtained paraffin-embedded block by a microtome. The obtained specimen section can be mounted on a slide, for example, by floating it on distilled water placed on the slide, warming and stretching it, and drying as it is, or by floating it on warm water and stretching it, and scooping it up with the slide.

In the separation step (3) in the method of the present invention, by separating part of the slide from the rest of the slide using an appropriate separation method, the specimen section mounted thereon can be separated simultaneously. As the separation method, each separation method described with respect to the slide of the present invention may be used. The area to be separated can be specified as a corresponding area by, for example, HE-staining one of the specimen-section-mounted slides obtained in the step (2) and marking a required area (for example, a cancer site).

In the step (4) of removing paraffin in the method of the present invention, the specimen section on the slide separated in the step (3) is deparaffinized. Examples of the deparaffinization treatment include, for example, a method using an organic solvent, a method using mineral oil, or the like.

In the deparaffinization treatment using an organic solvent, as the organic solvent, a solvent that does not dissolve the specimen section and can dissolve paraffin is used, and xylene can be exemplified. For example, when xylene is used, xylene is added to the slide on which the specimen section is mounted, mixed and centrifuged, and removed as the supernatant, to obtain the specimen section as a solid substance.

More particularly, a sufficient amount of xylene to soak the sample (1 mL of xylene) is added, and vortexed for 10 seconds. The mixture is centrifuged at room temperature (15 to 25°C) at 15000 rpm for 2 minutes to prepare deparaffinized cell pellets. After removing xylene as the supernatant, ethanol (96% to 100%) in the same amount (1 mL) as xylene is added, and vortexed for 10 seconds to extract residual xylene from the sample with ethanol. The mixture is centrifuged at room temperature at 15000 rpm for 2 minutes, and ethanol as the supernatant is removed to obtain a tissue piece that can be extracted with DNA.

In the deparaffinization treatment using mineral oil, a sufficient amount of mineral oil to soak the sample is added to the slide on which the specimen section is mounted, and mixed; a cell solubilizing aqueous solution containing protease is added, and heated (for example, at 56°C for 1 hour); and an aqueous phase of the two-phase separated aqueous phase/oil phase is recovered, to collect nucleic acid (DNA) in the specimen section.

More particularly, deparaffinization can be carried out by adding an appropriate amount (300 µL) of mineral oil and incubating at 90°C for 20 minutes, and after that, it can be extracted with a general DNA extraction kit (Analytical Biochemistry, 395 (2009), 265-267).

Furthermore, a deparaffinization reagent (Deparaffinization Solution; QIAGEN) is on the market as a product using the same principle. In this reagent, an appropriate amount (distinguished depending on the amount of the section: 160 µL or 320 µL) of Deparaffinization Solution can be added, vortexed for 10 seconds, and incubated at 56°C for 3 minutes, and the incubated sample can be returned to room temperature, and extracted using a general DNA extraction kit.

For example, according to an extraction kit manufactured by QIAGEN as an example of general extraction kits, Lysis buffer (180 µL) can be added, vortexed, and centrifuged, and then, Proteinase K (20 µL) can be added, incubated at 56°C for 1 hour, incubated at 90°C for 1 hour, and centrifuged, and an aqueous phase as the lower layer can be collected and treated using the extraction kit.

The specimen section obtained by the pretreatment method of the present invention, or the nucleic acid solution derived from the specimen section can be used as a sample for genetic testing in accordance with conventional methods.

### EXAMPLES

The present invention will now be further illustrated by, but is by no means limited to, the following Examples.

### <<Example 1: FFPE section fixation to polystyrene (PS) slides>>

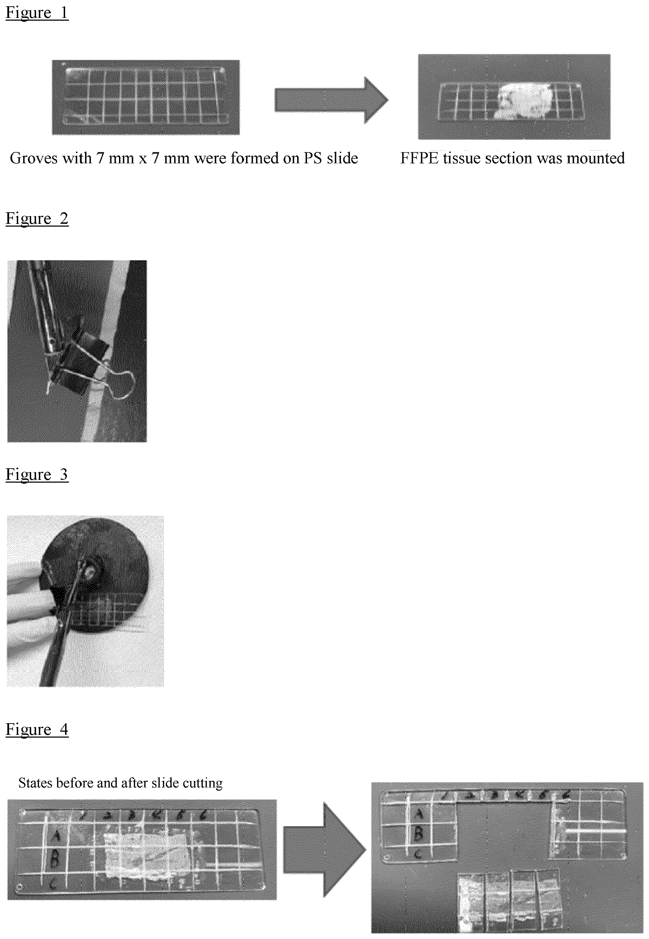

Lattice-like grooves were formed on one side of a PS slide (length 76 mm × width 26 mm × thickness 1 mm; KENIS) with a size of 7 mm × 7 mm (Fig. 1, left). A plastic cutter (Olfa) was used to form the grooves. The depth of the grooves was not been specified this time, but it was set to approximately 0.5 mm to 0.7 mm by visual estimation.

Formalin-fixed paraffin-embedded (FFPE) sections were fixed on slides in the same procedure as FFPE sections fixed on general microscope slides. First, an FFPE section cut out from a paraffin block to a thickness of 10 µm was floated in a water bath, and placed on the flat surface (a non-grooved side; a mounting surface) of the PS slide so as to be scooped out. After that, some water was removed by air-drying. Furthermore, in order to firmly adhere the FFPE section on the PS slide to the slide, with the grooved side (a basal surface) as the lower surface, the slide was placed on a heat block and heated at 65°C. Heating was carried out until paraffin became transparent (melted), and then naturally cooled to fix the FFPE section on the slide (Fig. 1, right).

### «Example 2: Cutting of polystyrene (PS) slides »

For cutting PS slides, a resin-cutting electric heating cutter (TAIYO ELECTRIC IND. CO., LTD.; HE-110) in which a spare blade of a scalpel was directly fixed was made by myself and used (Fig. 2). In connection with this, an ultrasonic cutter can also be used to cut the PS slide, and the slide can be easily cut. When an electric heating cutter is used, if it is brought into direct contact with the resin, the resin may melt and the slide may not be cut at the groove line. Therefore, in this Example, the resin was prevented from melting by cooling the slide from the lower surface of the slide (the side where the grooves were formed). While cooling the PS slide from below using an iron plate cooled at low temperature (-20°C), the slide was cut along the grooves from the upper surface of the slide (the side where the grooves were not formed)(Fig. 3). Fig. 4 shows a slide cut along the grooves.

### <<Example 3: Examination of DNA extraction from polystyrene (PS) slides 1>>

Deparaffinization Solution (QIAGEN) was used for a deparaffinization treatment. DNA extraction was carried out from the recovered solution after deparaffinization using a QIAamp DNA FFPE Tissue kit (QIAGEN). The treatment was carried out according to the procedure described in the package insert. Subsequent examination of DNA extraction was carried out according to the following procedure, unless otherwise specified:
(1) Add 320 µL of Deparaffinization Solution.
(2) Incubate at 56°C for 3 min., and then allow to cool at room temperature.
(3) Add 180µL of a Lysis buffer (Buffer ATL; QIAGEN), and mix by vortexing. Centrifuge for 1 min at 10,000 rpm.
(4) Add 20 µL of proteinase K (QIAGEN).
(5) Incubate at 56°C for 1 hour in a shaking heat block.
(6) Incubate at 90°C for 1 hour in a heat block.
(7) Add 200 µL of a kit solution (Buffer AL; QIAGEN).
(8) Add 200 µL of ethanol.
(9) Transfer the lower, liquid phase.
(10) Carry out purification using a QIAamp MinElute Column.

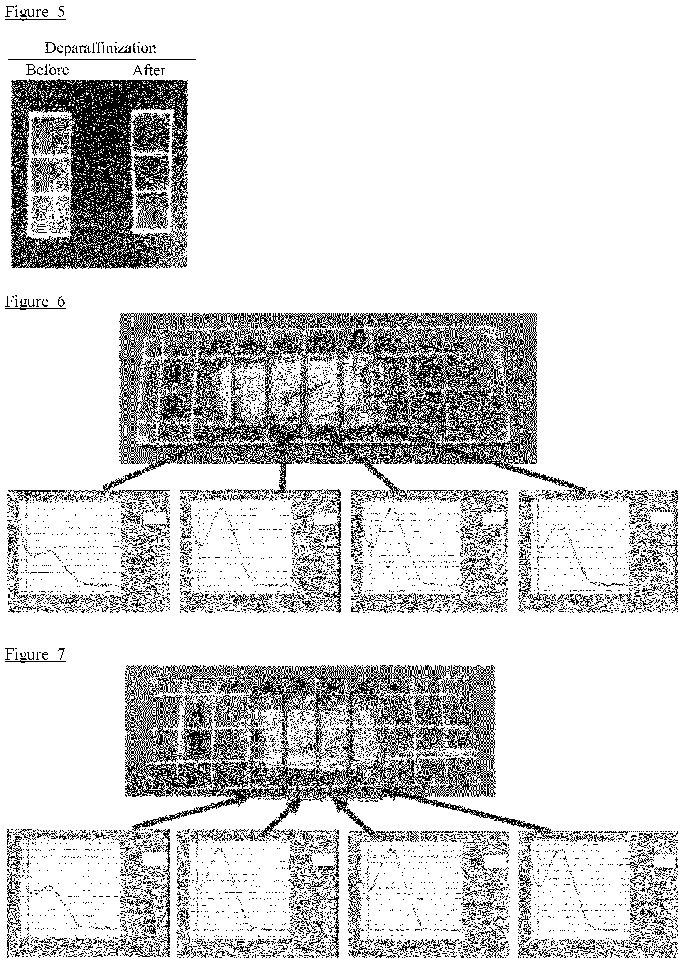

The purpose of this Example was to confirm whether the FFPE sections could be recovered in a solution, and to confirm the change of the PS slide pieces due to the treatment. Each PS slide piece (slide pieces 2, 3, 4, and 5) after cutting, as shown in Fig. 4, was placed in a 2 mL microtube (Eppendorf tube), and DNA extraction was carried out according to the procedure described in the package insert. After the above step (4), it was confirmed that the FFPE sections fixed on the slide were peeled off cleanly (Fig. 5). When the heat treatment at 90°C in the above step (5) was carried out, the PS slide pieces became milky white, but the shape remained.

### <<Example 4: Examination of DNA extraction from polystyrene (PS) slides 2>>

The purpose of this Example was to confirm whether there was no effect on DNA extraction.

As in Example 3, using each PS slide pieces (slide pieces 2, 3, 4, and 5) shown in FIGS. 6 and 7, DNA extraction was carried out from the recovered solutions after deparaffinization using a QIAamp DNA FFPE Tissue kit (QIAGEN). The DNA extraction was carried out according to the procedure described in the package insert.

DNA was eluted using 30 µL of an eluent, and the DNA concentration after elution was measured using a NanoDrop microvolume spectrophotometer (Thermo Fisher Scientific). The results of extraction from 2 slide pieces of 7 mm × 7 mm (Fig. 6) and extraction from 3 slide pieces of 7 mm × 7 mm (Fig. 7) are shown.

Furthermore, the amount of DNA per tissue volume was estimated from the amount of DNA extracted this time. The results are shown in Table 1. The tissue was not fixed on the entire surface of the slide used for extraction, but for the purpose of extrapolation, the tissue area was calculated as the area (7 mm × 7 mm) of the slide used this time. That is to say, in the case of 2 areas, it was calculated as 7 mm × 7 mm × 2 areas × 10 µm (FFPE section thickness), and in the case of 3 areas, it was calculated as 7 mm × 7 mm × 3 areas × 10 µm (FFPE section thickness), respectively. From the calculated results, the same amounts were extracted as the results (Empirical data 3: DNA yields from FFPE blocks produced in routine clinical practice: median approximately 0.5 µg/mm³ in surgical specimens, median approximately 2 µg/mm³ in biopsy specimens) described in "The Guidelines on the Handling of Pathological Tissue Samples for Genomic Medicine (Genome Shinryo-yo Byori Soshiki Kentai Toriatsukai Kitei)" published by The Japanese Society of Pathology on March 1, 2018. From this, it was confirmed that the same or greater amount of DNA as the conventional extraction process could be extracted by the method of the present invention.

**[Table 1]**

| Area | Extraction concentration (ng/µL) | Amount of DNA (µg) | DNA amount per tissue volume (µg/mm³) |
|---|---|---|---|
| 2-AB | 26 | 0.79 | 0.80 |
| 3-AB | 110 | 3.30 | 3.37 |
| 4-AB | 129 | 3.86 | 3.94 |
| 5-AB | 55 | 1.65 | 1.68 |
| 2-ABC | 32 | 0.96 | 0.65 |
| 3-ABC | 128 | 3.85 | 2.62 |
| 4-ABC | 187 | 5.60 | 3.82 |
| 5-ABC | 122 | 3.65 | 2.48 |

### <<Example 5: Evaluation of extracted DNA>>

In this Example, it was evaluated whether the gene of interest could be amplified from the extracted DNAs by real-time PCR measurement. The target of amplification is an RHOA gene, and the sequences of primers and a probe used for the measurement are shown in Table 2.

**[Table 2]**

| Type | Sequence | SEQ ID NO: |
|---|---|---|
| F primer | 5'-cctatgacttcttgtgcattgc-3' | 1 |
| R primer | 5'-tacacctctgggaactggtc-3' | 2 |
| Probe | 5'-gctgccatccggaagaaactg-3' | 3 |

The reaction composition and reaction temperature of real-time PCR are shown below.

The measurement was carried out using 200 nmol/L forward primer (F primer), 200 nmol/L reverse primer (R primer), 100 nmol/L probe, and Premix Ex taq (Probe qPCR) Master Mix (Takara). As samples, 5 µL of samples of each concentration shown in Table 1 were used for the measurement. The real-time PCR measurement was carried out using LC480 (Roche). For the reaction temperature, after incubating at 95°C for 30 seconds, 45 cycles consisting of incubations at 95°C for 3 seconds and at 62°C for 30 Seconds were carried out. For comparison of resin slide extracts, an FFPE section extracted from a microscope slide by the conventional method was used.

As a result, the DNAs extracted in Fig. 6 were 115% on average, with respect to the extraction from the conventional microscope slide. Furthermore, the DNAs extracted in Fig. 7 were 95% on average. From this, when real-time PCR was carried out, no effect was observed between the DNA extracted from the conventional microscope slide and the DNA extracted by the method of the present invention.

### INDUSTRIAL APPLICABILITY

The present invention can be used in the field of genetic testing.

Although the present invention has been described with reference to specific embodiments, various changes and modifications obvious to those skilled in the art are possible without departing from the scope of the appended claims.

### FREE TEXT IN SEQUENCE LISTING

SEQ ID NOs: 1 to 3 in the Sequence Listing are F primer, R primer, and Probe, respectively.

## Claims

1. A specimen slide for genetic testing on which a specimen section is to be mounted, wherein part of the slide can be separated, together with the specimen section to be separated, from the rest of the slide, while maintaining an upper surface and a lower surface of the slide.

2. A specimen slide for genetic testing on which a specimen section is to be mounted, wherein a guide that assists in separating part of the slide from the rest of the slide is disposed on a surface on which the specimen section is to be mounted and/or an opposite surface thereof.

3. A specimen slide for genetic testing on which a specimen section is to be mounted, wherein a groove that assists in separating part of the slide from the rest of the slide is disposed on an opposite surface of a surface on which the specimen section is to be mounted.

4. A system for pretreating a specimen, said system comprising:
an apparatus for pretreating a specimen equipped with a separator capable of separating part of the specimen slide for genetic testing according to any one of claims 1 to 3 from the rest of the slide; and
the specimen slide for genetic testing according to any one of claims 1 to 3.

5. A method of pretreating a specimen, said method comprising:
(1) preparing a specimen section;
(2) mounting the specimen section on a slide; and
(3) separating part of the slide, together with the specimen section mounted on the area, from the rest of the slide.

6. A method of pretreating a specimen, said method comprising:
(1) preparing a paraffin-embedded specimen section;
(2) mounting the specimen section on a slide;
(3) separating part of the slide, together with the specimen section mounted on the area, from the rest of the slide; and
(4) removing paraffin from the specimen section on the separated slide.
